# EUROPEAN PATENT SPECIFICATION

(11) **EP 2 730 299 B1**
(45) Date of publication and mention of the grant of the patent: **18.09.2019**
(21) Application number: 12191874.2
(22) Date of filing: 08.11.2012
(51) Int. Cl.: A61M 1/00

(54) **Lid for container for collecting organic liquids, adapter for the connection to said lid, kit comprising said lid and said adapter and container for collecting organic liquids comprising said lid**
Deckel für einen Behälter zum Sammeln organischer Flüssigkeiten, Adapter zum Verbinden mit dem Deckel, Satz mit dem Deckel und dem Adapter, und Behälter zum Sammeln organischer Flüssigkeiten mit dem Deckel
Couvercle pour récipient permettant de recueillir des liquides organiques, adaptateur pour la connexion audit couvercle, kit comprenant ledit couvercle et ledit adaptateur et récipient permettant de recueillir des liquides organiques comprenant ledit couvercle

(43) Date of publication of application: 14.05.2014
(73) Proprietor: Flow Meter S.p.a., 24040 Levate (Bergamo) (IT)
(72) Inventor: Paratico, Roberto, 24040 Levate (Bergamo) (IT)
(74) Representative: Botti, Mario

(56) References cited:
- EP-A1- 1 535 634
- WO-A1-91/12033
- WO-A1-2012/087376
- US-A- 5 901 717
- US-A1- 2008 284 167

## Description

### Field of application

The present invention relates in particular to a container for collecting organic liquids removed by aspiration from patients and/or from disabled persons. The invention also relates to a kit comprising lid and adapter, and to the container itself for collecting organic liquids.

The containers of the aforementioned type have a useful application in medical and paramedical environments.

### Prior Art

Containers for collecting organic liquids removed by aspiration from patients and/or invalid persons, comprising a cup and a lid hermetically closed onto the mouth of the said cup, are known.

These containers are present on the market in two different versions. In a first version, the container is of the disposable type and collects the removed organic liquids directly inside the rigid cup. In a second version, a soft collection bag fixed to the lid is instead provided; in this case the cup may be used again by replacing in each case the lid with associated bag, which is sold separately.

The lid is provided with at least two ports for connection, on one side, to a tube to be connected to a vacuum source (vacuum port) and, on the other side, to an aspiration tube for removing the organic liquids from a patient (patient port). The vacuum source may consist of a hospital vacuum plant or a dedicated vacuum pump.

Both the ports communicate directly with the inside of the cup or the collection bag when provided; moreover, in the version with collection bag, the vacuum port is connected also to the interspace between bag and cup so as to prevent the bag itself from collapsing.

Both the ports have externally a nozzle which receives, fitted thereon, the flexible tube respectively connected to the vacuum source or to the liquid aspiration needle. It should be noted that the two tubes used generally have a diameter which is substantially the same.

When the vacuum source is activated, the container is placed under a vacuum via the vacuum port and the organic liquids are removed via the patient port.

In order to prevent the entry of organic liquids and possible bacterial contamination of the vacuum generating system, whether it be a hospital plant or a single pump, it is of fundamental importance to provide a filter or overflow valve for protecting the vacuum port of the collection container. In the technologically more advanced embodiments, this filter consists of a water-repellent membrane filter which interrupts the suction flow when it is reaches a maximum permitted level, ensuring at the same time the bacterial and viral protection of the hospital vacuum plant.

Containers according to the above discussed prior art are disclosed for instance in documents WO 91/12033 A1 and EP 1 535 634 A1.

Although substantially satisfying the requirements of the sector in which they are used, the containers for collecting liquids according to the prior art nevertheless have a major drawback which has been noted by the proprietor of the present application and which is described below.

It happens that medical and paramedical staff, through lack of experience or oversight, may invert the connections of the aforementioned collection container. In fact since the aspiration tube and the vacuum source connection tube have the same diameter, it may happen that, by mistake, the former may be fitted onto the vacuum port nozzle and the latter on the patient port nozzle.

The abovementioned connection error may seem like a banal error which is unlikely since the two ports can be easily differentiated by a qualified person and have different forms, colours and writing. On the other hand, however, it must also be considered that the connection operation must be repeated by paramedical personnel whenever the container is filled and often under conditions of great psychological pressure due, for example, to the urgent nature of the action required. The frequent repetition of the operation and the not always optimum psychophysical conditions of the staff involved therefore explain the reason why the aforementioned connection error has an incidence rate which is not equal to zero (albeit marginally).

Although infrequent, the connection error described above has serious consequences which justify the attempt to find a suitable solution. When, in fact, the connection of the ports is inverted, not only does the bag, if present, collapse, but organic liquids enter via the vacuum port. The liquids thus force the protection filter and may flow back up through the patient port towards the suction plant (or associated machine). The error therefore results above all in damage to the collection container, but in particular in a major risk of contamination of the vacuum plant and the directly connected suction regulation devices, with the subsequent need for long and costly cleaning and disinfection operations.

A container comprising a lid with a vacuum port is disclosed in document US 5,901,717, wherein a port-specific adapter for the vacuum line is provided. However, the system does not fully prevent the possibility of the previously described connection error.

The problem of the present invention is therefore that of providing a device which is able to solve the drawback noted in the prior art, namely that of eliminating totally or reducing significantly the possibility of damage to the filter and contamination of the vacuum source, which may be due to lack of experience or an oversight on the part of the healthcare staff.

### Summary of the invention

The abovementioned technical problem is solved in particular by a lid for a container for collecting organic liquids, designed to close sealingly a cup of said container for collecting organic liquids. The lid comprises a patient port for the connection to an aspiration tube for removing the organic liquids from a patient and a vacuum port for connection to the vacuum source connection tube. The vacuum port is specifically formed so that it can be connected to a corresponding adapter associable with one end of said vacuum source connection tube.

Said vacuum port comprises a connecting member which defines a female connection.

The presence of a female connection makes it technically impossible to connect the free end of the aspiration tube, which will thus be correctly engaged with the nozzle provided on the patient port.

The aforementioned female connection is in particular a conical connection, in particular advantageous since it allows easy and efficient sealed insertion of the specific adapter.

The aforementioned lid, owing to its innovative technical characteristics, eliminates the possibility of a critical error in the connection of the connection tubes. It is in fact impossible for an inexpert or absent-minded healthcare worker to connect the aspiration tube for removing the organic liquids to the vacuum port. This tube is in fact certainly without the specific adapter which allows connection to the vacuum port.

The specific adapter will instead be stably associated with the connection end of the vacuum source connection tube, such that the operator, even if not possessing suitable training, will obviously be induced to insert it in the correct position. It should be noted that fitting of the adapter to the connection tube must not be repeated every time the fluid collection container is connected, but must be performed once only. The fitting operation is therefore not of a repetitive nature which multiplies the possibility of error during the operations for connecting the collection containers provided with lids according to the prior art.

It should be noted, moreover, that any incorrect connection of the vacuum source tube without an adapter to the patient port, which may still occur with the lid according to the present invention, does not involve per se any risk of damage to the system, there being no connection of the vacuum port to an aspiration tube.

A person skilled in the art will understand how the lid comprises all the necessary features for solving the technical problem mentioned above, being able to be sold separately and applied to the cups of containers for collecting organic liquids, already present on the market.

Said vacuum port and said patient port may advantageously have different colours, where the adapter has a colour the same as or similar to that of the vacuum port. Thus for example, depending on a colour code which is recognized in the sector, both the vacuum port and the adapter may be coloured yellow, this colour indicating vacuum systems. The patient port may instead be coloured white.

The same colour of the vacuum port and the adapter clearly helps make the lid connection operation obvious and error-proof, such that it may be performed also by personnel without specific training.

To prevent any possibility of a wrong connection, the connecting member will have, at the inlet mouth of said female connection, an external diameter substantially greater than the internal diameter of the aspiration tubes typically used in the sector.

In particular, since said tubes generally have an internal diameter of 7 mm, it is proposed providing the connecting member with an external diameter not compatible with this dimension, for example greater than 10 mm.

Other systems may obviously be used in order to prevent the engagement of the end of a tube onto the connection of the vacuum port; for example the connecting member could have an external profile which is not circular.

The connecting member has preferably a tubular shape and is longitudinally inserted inside a corresponding engaging seat of a main body of said lid, said engaging seat having a communication opening which allows flow communication between said connecting member and the inside of the container for collecting organic liquids when said lid is sealingly mounted on the cup.

The structure described above has a plurality of advantages associated in particular with the reliability and simplicity of production of the resultant device. It should be noted in particular that the connecting member may be made by means of an injection-moulding process which is separate from that of the main body. This allows easy definition of the relatively complex forms of the internal fluid passage and the manufacture of the connecting member in plastic having a colour which is different from that of the main body.

Although manufactured separately from the main body of the lid, the connecting member is preferably locked in a non-removable manner inside said engaging seat.

The connecting member may moreover be further fastened to the main body by a ring, which is arranged aligned with the engaging seat and in a spaced relationship with respect thereto.

The connecting member may advantageously comprise an engaging portion designed to be inserted inside the engaging seat and a head portion, of larger diameter, defining internally the female connection. The greater diameter of the head portion defines a shoulder which may be arranged against the periphery of the aforementioned ring.

In particular, this engaging portion may project radially in relation to the perimeter of said main body, so as to allow easy engagement of the connection tube provided with adapter.

The lid is preferably provided with a filter or valve which is internally arranged so as to protect the vacuum port. This component part may be a mechanical overflow valve, a water-repellent backflow prevention filter, a membrane filter, an anti-bacterial filter or a combination of the above. Other component parts with a similar function may replace those parts mentioned above or include some of their features.

The lid may comprise a soft bag which is fixed to an inner surface thereof, said vacuum port and said patient port being in flow communication with the inside of said soft bag, said vacuum port being moreover in flow communication with an auxiliary opening which opens out on said inner surface of said lid in an external position with respect to said soft bag.

The present invention also relates to a kit which advantageously comprises both the lid described above and a corresponding adapter.

The adapter is associable with one end of a vacuum source connection tube, and it is specifically formed such that it can be connected to the vacuum port of a lid according to the present invention so as to allow flow communication between the vacuum source connection tube and said vacuum port.

The adapter may in particular comprise a connection portion which defines a male connection designed to be connected to said vacuum port and an oppositely arranged portion for attaching the vacuum source connection tube.

The attachment portion may have a greater length than the connection portion, thus defining a tapered nozzle onto which the vacuum source connection tube may be fitted.

This kit may be sold separately so as to supply users who already use containers for collecting organic liquids according to the prior art.

The aforementioned technical problem is also solved by a container for collecting organic liquids comprising a lid according to the present invention and a respective cup, said lid sealingly closing said cup, and said patient port and said vacuum port establishing flow communication between the outside and inside of said container for collecting organic liquids.

Further characteristic features and advantages of the invention will emerge from the description, provided hereinbelow, of an example of embodiment, provided by way of a non-limiting example, with reference to the accompanying drawings.

### Brief description of the drawings

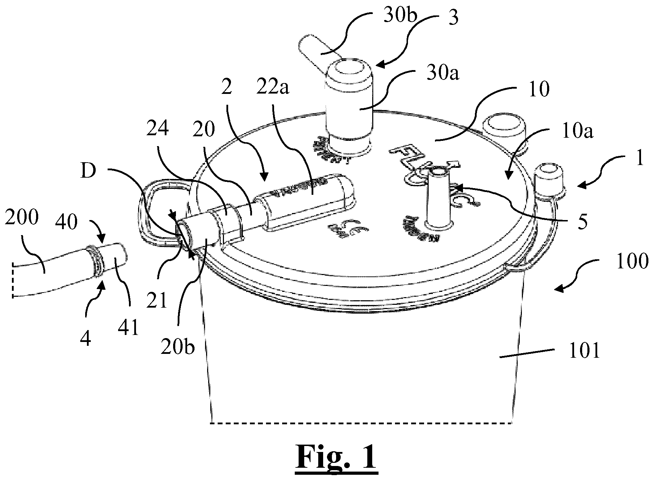

Figure 1 shows a perspective view of a container for collecting organic liquids with a lid according to the present invention connected and a vacuum source connection tube provided with a specific adapter, in a disconnected configuration;

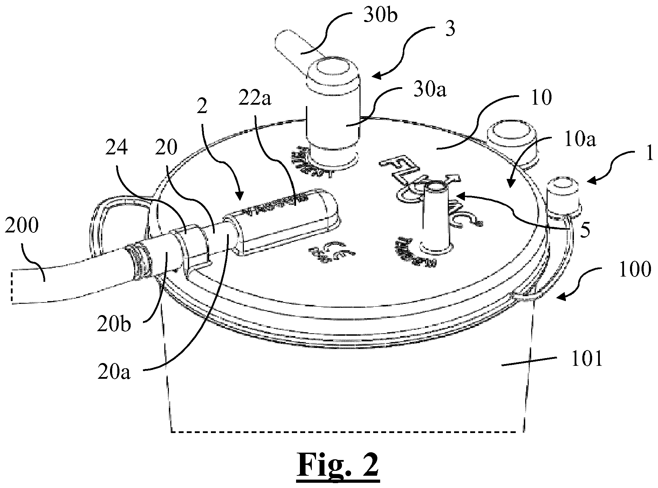

Figure 2 shows a perspective view of the parts according to Figure 1 in a connected configuration;

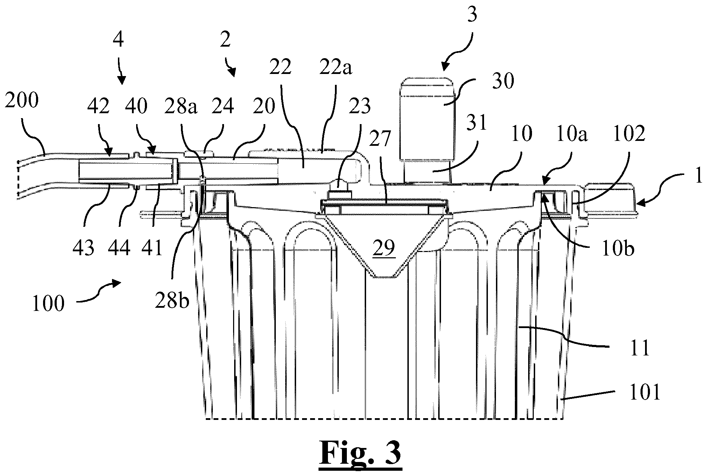

Figure 3 shows a cross-sectional side view, along a diametral plane, of the parts shown in Figure 2.

### Detailed description

With reference to the accompanying figures, 100 denotes generically a container according to the invention for collecting organic liquids removed by aspiration from patients and/or from disabled persons.

The container 100 comprises a cup 101 and a lid 1 according to the present invention which has a substantially circular shape and is hermetically fixed to the mouth of the said cup 101.

It should be noted that the container 100 and the lid 1 shown in the figures and described below relate to an embodiment where a soft bag 11 is fixed to an inner surface of the lid 1 and allows the cup 101 to be reused. The present invention may, however, also be applied equally well to disposable devices comprising a lid stably fixed onto the container without the presence of an internal bag.

As mentioned above, the lid 1 is hermetically and stably fastened, by means of peripheral fixing means 102 of the known type, on top of the cup 101. Since the cup 101 is substantially of the known type, the following description will concentrate mainly on the lid 1.

The lid 1 comprises a main body 10 with a substantially disk-like shape which has a top outer surface 10a and a bottom inner surface 10b.

The inner surface 10b, facing the inside of the cup 101, has, fixed thereto, a soft bag 11 which is designed to collect the organic liquids removed from the patient.

The outer surface 10a is provided with two connection ports, i.e. a vacuum port 2 and the patient port 3, both of which are defined by the main body 10 in combination with a corresponding connecting member 20, 30.

The main body 10 is made by means of moulding of a plastic, preferably having a neutral colour different from the colours used for the connecting members, for example blue. The connecting members 20, 30 are also separately made by means of plastic moulding and then associated with the main body 10. The connecting member 20 of the vacuum port 2 is preferably made of yellow plastic, while the connecting member 30 of the patient port 3 is preferably made of white plastic.

The patient port 3, designed to be associated with an aspiration tube (not shown in the attached figures) for removing the organic liquids from a patient, communicates with the inside of the soft bag 11 defined above. The vacuum port 2, designed to be associated with a vacuum source connection tube 200, communicates instead both with the inside of said soft bag 11 and with the interspace present between soft bag 11 and cup 101.

The patient port has a vertical sleeve 31, only partially visible in the figures, which is formed as one piece on the outer surface 10a of the main body 10 and which opens out into the soft bag 11.

The aforementioned connecting member 30 is fitted on top of said vertical sleeve 31 and has a cup-like portion 30a intended to be positioned over the vertical sleeve in flow communication with a tapered nozzle 30b which extends radially from the cup-like portion 30a. The free end of the aspiration tube for removing the organic liquids from the patient is fitted onto the nozzle 30b.

The vacuum port 2 has a horizontal axis and comprises, as mentioned above, a tubular-shaped connecting member 20.

The tubular connecting member 20 comprises two portions which are arranged coaxially in sequence: an engaging portion 20a, which is longer and has a smaller diameter, and a head portion 20b which is shorter and has a larger diameter.

A female conical connection 21 is defined inside the head portion and is formed so that it can be sealingly connected to a specific adapter 4 associable with the end of the connection tube 200 defined above.

The tubular-shaped adapter 4 has two portions which are arranged coaxially in sequence: i.e. it comprises a connection portion 40 and an attachment portion 42, which are separated from each other by a ring 44 projecting from their outer surface.

The connection portion 40 defines a male conical connection 41 which is shaped so as to match the female conical connection 21 previously introduced and is designed be to sealingly connected thereto.

The attachment portion 42, which has a length greater than the connection portion 40, defines a tapered nozzle 43 onto which the free end of the vacuum source connection tube 200 is fitted.

With reference again to the connecting member 20 of the vacuum port 2, it should be noted that the external diameter D of the head portion 20b of the connecting member 20 is advantageously greater than 10 mm, such that a standard connecting tube, with an internal diameter of 8 mm, may not be fitted onto this free end of the connecting member.

The other end of the connecting member 20 is inserted longitudinally inside a horizontal engaging seat 22 which is defined inside a radial sleeve 22a which protrudes upwards from the outer surface 10a of the main body 10. The engaging seat 22 defines an internal fluid path which connects the end of the connecting member 20 inserted inside it to a communication opening 23 which opens out on the inner surface 10b of the main body, inside the soft bag 11.

It should be noted that the communication opening 23 is internally protected by a membrane filter 27 which interrupts the suction flow when the maximum permitted level is reached, ensuring at the same time the bacterial protection of the hospital vacuum plant associated with the vacuum port 2. The membrane filter 27 is in turn mechanically protected by a spray-protection funnel 29.

The main body 10 is also provided on its outer surface 10a and in the region of its external circumference with a ring 24 which is coaxial with the engaging seat 22 and situated at a distance from the inlet mouth of the latter. The ring 24 is also passed through by the engaging portion 20a of the connecting member 20 which is then inserted inside the engaging seat 22.

The shoulder defined between engaging portion 20a and head portion 20b of the connecting member 20 bears against the outer face of the ring 24; the head portion 20b is thus extended radially so as to project in relation to the outer perimeter of the main body 10 of the lid 1.

The section of the engaging portion 20a which is embraced by the ring 24 has a radial through-hole 28a which is aligned with an auxiliary opening 28b formed in the main body 10 so as to connect the area situated between the inside of the cup 101 and the outside of the soft bag 11 to the vacuum source.

It should be noted that the connecting member 20 described above is mechanically fastened to the engaging seat 22 and the ring 24. In particular, said connecting member 20 cannot be extracted or rotated about its longitudinal axis.

The flow communication which is established between the vacuum port 2 and the soft bag 11 allows a vacuum to be created inside the latter when the vacuum source is activated.

Moreover, owing to the presence of the radial through-hole 28a and the auxiliary opening 28b, an equilibrium in the suction flow between the inside of the soft bag 11 and the inside of the cup 101 is created, allowing stretching of the said soft bag 11 over the inner walls of the cup 101.

The liquid is thus collected directly inside the soft bag 11. The latter and the lid 1 are disposed of after use while the cup 101, which does not come into contact with the liquids, may be reused.

The lid 1 is also provided with a third sleeve-like port 5 which opens out towards the inside of the cup 101 and allows the connection, in series, of several containers 100 which are identical to each other in a manner known per se.

Obviously, a person skilled in the art, in order to satisfy any specific requirements which arise, may make numerous modifications and variations to the invention described above, all of these being moreover contained within the scope of protection of the invention, as defined by the following claims.

## Claims

1. Lid (1) of a container for collecting organic liquids (100), which is designed to sealingly close a cup (101) of said container for collecting organic liquids (100), comprising a patient port (3) for connection to a an aspiration tube for removing the organic liquids from a patient and a vacuum port (2) for connection to a vacuum source connection tube (200), said vacuum port (2) comprising a connecting member (20); **characterized in that** said connecting member (20) defines a conical female connection (21), said vacuum port (2) being connectable to a corresponding adapter (4) associable with an end of said vacuum source connection tube (200).

2. Lid (1) according to Claim 1, wherein said connecting member (20) has, at the inlet mouth of said female connection (21), an external diameter (D) greater than 10 mm.

3. Lid (1) according to Claim 1, wherein said connecting member (20) has a tubular form and is longitudinally inserted inside a corresponding engaging seat (22) of a main body (10) of said lid (1), said engaging seat (22) having a communication opening (23) which allows flow communication between said connecting member (20) and the inside of the container for collecting organic liquids (100) when said lid is sealingly mounted on the cup (101).

4. Lid (1) according to Claim 3, wherein said connecting member (20) is furthermore fastened to the main body (10) by a ring (24), which is arranged aligned with the engaging seat (22) and in a spaced relationship with respect to the latter.

5. Lid (1) according to one of Claims 3 or 4, wherein said connecting member (20) comprises an engaging portion (20a) designed to be inserted inside the engaging seat (22) and a head portion (20b), of larger diameter, internally defining the female connection (21).

6. Lid (1) according to Claim 5, wherein said engaging portion (20a) projects radially in relation to the perimeter of said main body (10).

7. Lid (1) according to one of the preceding claims, comprising at least one filter (27) or valve which is internally arranged so as to protect the vacuum port (2).

8. Lid (1) according to one of the preceding claims, further comprising a soft bag (11) which is fixed to an inner surface (1Oa) of said lid (1), said vacuum port (2) and said patient port (3) being in flow communication with the inside of said soft bag (11), said vacuum port (2) being moreover in flow communication with an auxiliary opening (28b) which opens out on said inner surface (10b) of said lid (1) in an external position with respect to said soft bag (11).

9. Kit (1, 4) comprising a lid (1) according to one of Claims 1 to 8 and a corresponding adapter (4,) said adapter being specifically shaped such that it can be connected to the vacuum port (2) of the lid (1) so as to allow flow communication between the vacuum source connection tube (200) and said vacuum port (2), the adapter (4) comprising a connection portion (40) which defines a male connection (41) connectable to the vacuum port (2) and an oppositely arranged portion (42) for attachment of the vacuum source connection tube (200).

10. Kit (1, 4) according to Claim 9, wherein said vacuum port (2) and said patient port (3) have different colours, said adapter (4) having a colour similar to that of the vacuum port (2).

11. Container for collecting organic liquids (100) comprising a lid (1) according to one of Claims 1 to 8 and a cup (101), said lid (1) sealingly closing said cup (101), said patient port (3) and said vacuum port (2) allowing flow communication between the inside and outside of said container for collecting organic liquids (100).

## Patentansprüche

1. Deckel (1) eines Behälters zum Sammeln organischer Flüssigkeiten (100), der dazu bestimmt ist, einen Becher (101) des genannten Behälters zum Sammeln organischer Flüssigkeiten (100) dicht zu verschließen, umfassend eine Patientenöffnung (3) zum Anschluss an ein Ansaugrohr zum Entfernen der organischen Flüssigkeiten von einem Patienten und eine Vakuumöffnung (2) zum Anschluss an ein Vakuumquellenverbindungsrohr (200), wobei die genannte Vakuumöffnung (2) ein Verbindungselement (20) umfasst;
**dadurch gekennzeichnet, dass** das genannte Verbindungselement (20) eine konische weibliche Verbindung (21) definiert, dass die genannte Vakuumöffnung (2) mit einem entsprechenden Adapter (4) verbindbar ist, der mit einem Ende des genannten Vakuumquellenverbindungsrohrs (200) verbunden werden kann.

2. Deckel (1) nach Anspruch 1, wobei das Verbindungselement (20) an der Einlassöffnung der weiblichen Verbindung (21) einen Außendurchmesser (D) von mehr als 10 mm aufweist.

3. Deckel (1) nach Anspruch 1, wobei das genannte Verbindungselement (20) eine rohrförmige Form aufweist und in Längsrichtung in einen entsprechenden Eingriffssitz (22) eines Hauptkörpers (10) des genannten Deckels (1) eingesetzt ist, wobei dieser Eingriffssitz (22) eine Verbindungsöffnung (23) aufweist, die eine Strömungsverbindung zwischen dem genannten Verbindungselement (20) und der Innenseite des Behälters zum Sammeln organischer Flüssigkeiten (100) ermöglicht, wenn der genannte Deckel dicht auf dem Becher (101) montiert ist.

4. Deckel (1) nach Anspruch 3, wobei das genannte Verbindungselement (20) ferner am Hauptkörper (10) durch einen Ring (24) befestigt ist, der mit dem Eingriffssitz (22) ausgerichtet und in einer beabstandeten Beziehung zu diesem angeordnet ist.

5. Deckel (1) nach einem der Ansprüche 3 oder 4, wobei das genannte Verbindungselement (20) einen Eingriffsabschnitt (20a), der dazu bestimmt ist, in den Eingriffssitz (22) eingeführt zu werden, und einen Kopfabschnitt (20b) mit größerem Durchmesser umfasst, der innen die weibliche Verbindung (21) definiert.

6. Deckel (1) nach Anspruch 5, wobei der genannte Eingriffsabschnitt (20a) radial in Bezug auf den Umfang des genannten Hauptkörpers (10) vorsteht.

7. Deckel (1) nach einem der vorstehenden Ansprüche, umfassend mindestens einen Filter (27) oder ein Ventil, das bzw. der intern so angeordnet ist, dass er die Vakuumöffnung (2) schützt.

8. Deckel (1) nach einem der vorhergehenden Ansprüche, ferner umfassend einen weichen Beutel (11), der an einer Innenfläche (10a) des genannten Deckels (1) befestigt ist, wobei die genannte Vakuumöffnung (2) und die genannte Patientenöffnung (3) in Strömungsverbindung mit der Innenseite des genannten weichen Beutels (11) stehen, wobei die genannte Vakuumöffnung (2) außerdem in Strömungsverbindung mit einer Hilfsöffnung (28b) steht, die sich auf der genannten Innenfläche (10b) des genannten Deckels (1) in einer äußeren Position in Bezug auf den genannten weichen Beutel (11) öffnet.

9. Bausatz (1, 4) mit einem Deckel (1) nach einem der Ansprüche 1 bis 8 und einem entsprechenden Adapter (4), wobei der genannte Adapter speziell so geformt ist, dass er mit der Vakuumöffnung (2) des Deckels (1) verbunden werden kann, um eine Strömungsverbindung zwischen dem Vakuumquellenverbindungsrohr (200) und der genannten Vakuumöffnung (2) zu ermöglichen, wobei der Adapter (4) einen Verbindungsabschnitt (40) umfasst, der eine männliche Verbindung (41), die mit der Vakuumöffnung (2) verbindbar ist, und einen gegenüberliegenden Abschnitt (42) zur Befestigung des Vakuumquellenverbindungsrohrs (200) definiert.

10. Bausatz (1, 4) nach Anspruch 9, wobei die genannte Vakuumöffnung (2) und die genannte Patientenöffnung (3) unterschiedliche Farben aufweisen, wobei der genannte Adapter (4) eine ähnliche Farbe wie die der Vakuumöffnung (2) aufweist.

11. Behälter zum Sammeln von organischen Flüssigkeiten (100), umfassend einen Deckel (1) nach einem der Ansprüche 1 bis 8 und einen Becher (101), wobei der genannte Deckel (1) den genannten Becher (101) abdichtend verschließt, wobei die genannte Patientenöffnung (3) und die genannte Vakuumöffnung (2) eine Strömungsverbindung zwischen der Innenseite und der Außenseite des genannten Behälters zum Sammeln von organischen Flüssigkeiten (100) ermöglichen.

## Revendications

1. Couvercle (1) d'un contenant destiné à collecter des liquides organiques (100), qui est conçu pour fermer de façon étanche une coupe (101) dudit contenant destiné à collecter des liquides organiques (100), comprenant un orifice de patient (3) pour raccordement à un tube d'aspiration destiné à éliminer les liquides organiques d'un patient et un orifice à vide (2) pour raccordement à un tube de raccordement de source à vide (200), ledit orifice à vide (2) comprenant un organe de raccordement (20) ; **caractérisé en ce que** ledit organe de raccordement (20) définit un raccord femelle conique (21), ledit orifice à vide (2) pouvant être raccordé à un adaptateur correspondant (4) pouvant être associé à une extrémité dudit tube de raccordement de source à vide (200).

2. Couvercle (1) selon la revendication 1, dans lequel ledit organe de raccordement (20) a, au niveau de l'embouchure d'admission dudit raccordement femelle (21), un diamètre externe (D) plus grand que 10 mm.

3. Couvercle (1) selon la revendication 1, dans lequel ledit organe de raccordement (20) a une forme tubulaire et est inséré longitudinalement à l'intérieur d'une assise d'engagement (22) correspondante d'un corps principal (10) dudit couvercle (1), ladite assise d'engagement (22) ayant une ouverture de communication (23) qui permet une communication d'écoulement entre ledit organe de raccordement (20) et l'intérieur du contenant destiné à collecter des liquides organiques (100) lorsque ledit couvercle est monté de façon étanche sur la coupe (101).

4. Couvercle (1) selon la revendication 3, dans lequel ledit organe de raccordement (20) est de plus arrimé au corps principal (10) par une bague (24), qui est agencée alignée avec l'assise d'engagement (22) et dans une relation espacée par rapport à cette dernière.

5. Couvercle (1) selon l'une des revendications 3 ou 4, dans lequel ledit organe de raccordement (20) comprend une portion d'engagement (20a) conçue pour être insérée à l'intérieur de l'assise d'engagement (22) et une portion de tête (20b), de plus grand diamètre, définissant à l'intérieur le raccord femelle (21).

6. Couvercle (1) selon la revendication 5, dans lequel ladite portion d'engagement (20a) dépasse radialement vis-à-vis du périmètre dudit corps principal (10).

7. Couvercle (1) selon l'une des revendications précédentes, comprenant au moins un filtre (27) ou une valve qui est agencé(e) à l'intérieur de manière à protéger l'orifice à vide (2).

8. Couvercle (1) selon l'une des revendications précédentes, comprenant en outre un sac souple (11) qui est fixé à une surface interne (10a) dudit couvercle (1), ledit orifice à vide (2) et ledit orifice de patient (3) étant en communication d'écoulement avec l'intérieur dudit sac souple (11), ledit orifice à vide (2) étant de surcroît en communication d'écoulement avec une ouverture auxiliaire (28b) qui débouche sur ladite surface interne (10b) dudit couvercle (1) dans une position externe par rapport audit sac souple (11).

9. Kit (1, 4) comprenant un couvercle (1) selon l'une des revendications 1 à 8 et un adaptateur correspondant (4), ledit adaptateur étant spécifiquement conformé pour qu'il puisse être raccordé à l'orifice à vide (2) du couvercle (1) de manière à permettre une communication d'écoulement entre le tube de raccordement de source à vide (200) et ledit orifice à vide (2), l'adaptateur (4) comprenant une portion de raccordement (40) qui définit un raccord mâle (41) pouvant être raccordé à l'orifice à vide (2) et une portion agencée à l'opposé (42) pour attache du tube de raccordement de source à vide (200).

10. Kit (1, 4) selon la revendication 9, dans lequel ledit orifice à vide (2) et ledit orifice de patient (3) ont des couleurs différentes, ledit adaptateur (4) ayant une couleur similaire à celle de l'orifice à vide (2).

11. Contenant destiné à collecter des liquides organiques (100) comprenant un couvercle (1) selon l'une des revendications 1 à 8 et une coupe (101), ledit couvercle (1) fermant de façon étanche ladite coupe (101), ledit orifice de patient (3) et ledit orifice à vide (2) permettant une communication d'écoulement entre l'intérieur et l'extérieur dudit contenant destiné à collecter des liquides organiques (100).
